# EUROPEAN PATENT SPECIFICATION

(11) **EP 2 163 500 B1**
(45) Date of publication and mention of the grant of the patent: **07.05.2014**
(21) Application number: 09252173.1
(22) Date of filing: 14.09.2009
(51) Int. Cl.: B65H 45/00

(54) **Wipe assembly and method of cleaning using a wipe assembly**
Wischeinheit und Verfahren zur Reinigung unter Verwendung einer Wischeinheit
Assemblage de lingette et procédé de nettoyage utilisant un assemblage de lingette

(30) Priority: 15.09.2008 US 210682; 15.09.2008 US 210729
(43) Date of publication of application: 17.03.2010
(73) Proprietor: McNeil-PPC, Inc., Skillman, NJ 08558 (US)
(72) Inventor: Beatty, Heidi, Hillsborough, NJ 08844 (US); Tarazona, Luz Maria Romero, 35128 Padova (IT); Vassallo, Alessandro, 28440 Madrid (ES)
(74) Representative: Kirsch, Susan Edith

(56) References cited:
- CN-Y- 2 933 258
- JP-A- 10 066 671
- TW-U- M 332 466
- US-A- 1 643 722

## Description

### FIELD OF THE INVENTION

The present invention relates to a wipe assembly and a method of cleaning using a wipe assembly, and more particularly to a wipe assembly including a plurality of individual wipes arranged in a stacked configuration and a method of using such a wipe assembly.

### BACKGROUND OF THE INVENTION

Disposable personal care wipes, such as for example baby wipes or wet hand towelettes, are well known in the art and come in a variety of forms and configurations. Such personal wipes are often packaged in one of two common package types. The first package type consists of a liquid impermeable material, such as a metal foil, that contains an individual wipe therein. This type of package is commonly used for wet hand towelettes and the like. The second package type consists of a rigid container, such as a plastic container, that contains a plurality of individual wipes and permits to user to individually remove a single wipe for use. This type of package is commonly used for baby wipes and the like.

The inventor of the present invention has discovered that a common problem with prior art wipes of the type described above is that once the wipe is used to clean a surface the user must first dispose of the soiled wipe and then grasp a new clean wipe prior to further cleaning. That is, the user must interrupt the cleaning process to first dispose of the soiled wipe and then grasp a new clean wipe prior to continuing the cleaning process. The above described problem is further complicated by the fact that the user may have to open a new foil wrapper, in the case of a wet hand towelette, or may have to retrieve a new clean wipe from within a rigid container, in the case of a baby wipe, prior to further cleaning.

CN 2 933 258 discloses a wipe assembly comprising planar wipes which can be rotated over the top of a base of the wipe assembly to reveal an underlying wipe.

JP 10 066671 discloses a dustcloth having a front cover comprising an opening and a base, wherein a user is able to insert at least a portion of their hand through the opening and between the front cover and base.

US 1 643 722 discloses a polishing mitten having a plurality of nested bag-shaped polishing cloths embracing the mitten.

TW M 332 466 discloses a block of wipes. The bottom most wipe is used to clean and is then rotated over the top of the block of wipes, to expose the next wipe.

In view of the above, the inventor of the present invention has discovered a wipe assembly, and a method of cleaning using such a wipe assembly, that overcomes the shortcomings of the prior art wipes described above. In particular, the wipe assembly according to the present invention permits the sequential use of a plurality of clean wipes without significant interruption of the cleaning process.

### SUMMARY OF THE INVENTION

In view of the foregoing, the present invention provides, according to a first aspect of the invention, a wipe assembly as defined in claims 1-12.

The present invention provides, according to another aspect of the invention, a method of cleaning comprising the steps of: maintaining a wipe assembly on a palm side of a user's hand, the wipe assembly including a plurality of wipes arranged in a stacked configuration, each one of the wipes having a first and second opposed surfaces; cleaning a surface with a bottom wipe of the plurality of wipes; rotating the bottom wipe from a first position to a second position, wherein in the first position the second surface of the wipe is arranged in an outwardly facing configuration and in the second position the second surface of an underlying wipe is arranged in an outwardly facing configuration; manually retaining the bottom wipe in the second position; and cleaning a surface with the underlying wipe.

### BRIEF DESCRIPTION OF THE DRAWINGS

FIG. 1 is a perspective view of a wipe assembly according to a first embodiment of the present invention;
FIG. 2 is a perspective view of a wipe assembly shown in FIG.1 with a user's hand partially inserted within the wipe assembly;
FIG. 3 is a perspective view depicting a method for folding a substrate to form the wipe assembly shown in FIG. 1;
FIG. 4 is an exploded perspective view of a wipe assembly according to a second embodiment of the present invention;
FIGS. 5-8 depict a method of using the wipe assembly shown FIG. 1;
FIG. 9 is a perspective view of a wipe assembly according to a third embodiment of the present invention with a user's hand partially inserted within the wipe assembly; and
FIG. 10 is an exploded perspective view of the wipe assembly shown in FIG 9.

### DETAILED DESCRIPTION OF THE INVENTION

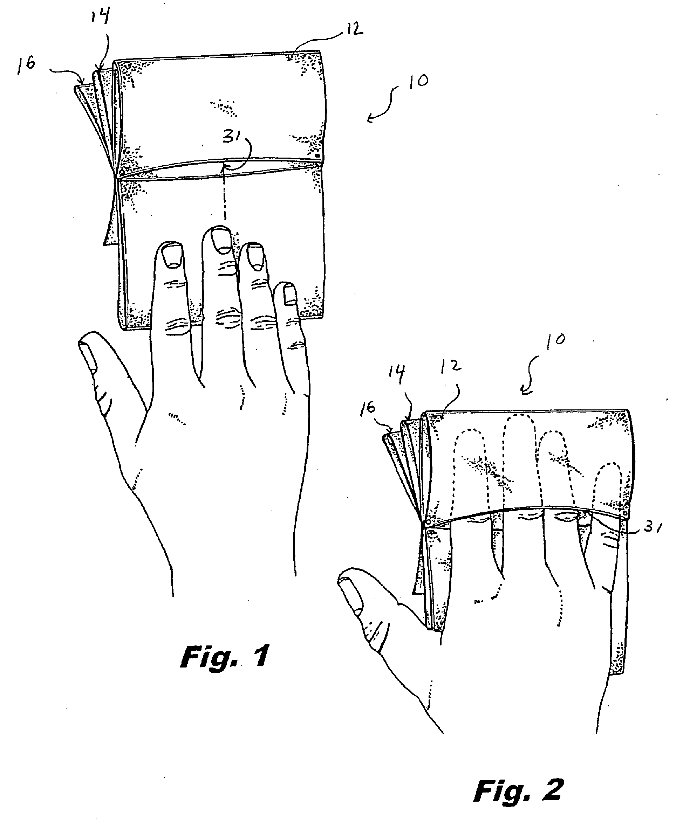

Referring to Fig. 1, a first embodiment of the wipe assembly 10 according to the present invention generally includes a top wipe 12, an intermediate wipe 14, and a bottom wipe 16. Each of the intermediate and bottom wipes 14, 16 are arranged below the top wipe 12. Each of the wipes 12, 14 and 16 are coupled to one another and are arranged in a "stacked configuration" as shown in Fig. 1. The term "stacked configuration" as used herein means that each of the wipes has a first position wherein the wipes are arranged in a substantially overlapping relationship.

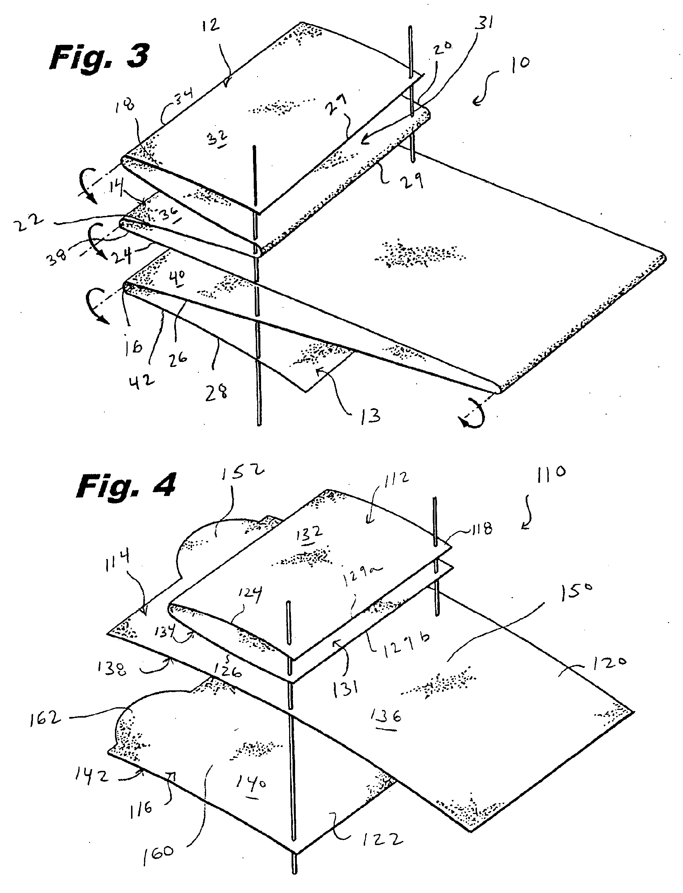

Referring to Fig. 3, the specific embodiment of the wipe assembly 10 shown in Figs. 1-3 is preferably constructed from a single sheet 13 of material that is folded in the manner shown in Fig. 3 to thereby form the individual wipes 12, 14 and 16. As shown in Fig. 3, each individual wipe 12, 14 and 16 is formed by folding the sheet 13 so that each wipe is defined by two overlapping layers of material. Specifically, the wipe 12 is defined by material layers 18 and 20, wipe 14 is defined by material layers 22 and 24, and wipe 16 is defined by material layers 26 and 28. The material layers 18, 20, 22, 24, 26 and 28 are then coupled to one another in the folded configuration shown in Fig. 3 to thereby form the final wipe assembly 10 shown in Figs. 1 and 2.

The layers 18 and 20 of the first wipe 12 are left in a detached state, i.e. they are not sealed to one another, along a proximal edge 29 thereof. In this manner, a chamber 31 is defined between the layers 18 and 20 of the first wipe 12. As shown in Figs. 1 and 2, the chamber 31 is structured and arranged to receive at least a portion of a user's hand during use. More specifically, the chamber 31 is structured and arranged to receive at least a portion of the user's fingers. In this manner, as shown in Figs. 1 and 2, a user can maintain the wipe assembly 10 on the palm side of the user's hand during use.

As shown in Fig. 3, the top wipe 12 includes a first surface 32 and a second opposed surface 34. Likewise, the intermediate wipe 14 includes opposed surfaces 36 and 38 and the bottom wipe 16 includes opposed surfaces 40 and 42. Although the specific embodiment of the wipe assembly 10 shown in Figs. 1-3 includes three individual wipes, the wipe assembly 10 according to the present invention may include two or more individual wipes.

Although the wipe assembly 10 shown in Figs. 1-3 is constructed from a single folded sheet of material it is possible that the wipe assembly 10 could be constructed from distinct layers of material that are coupled to one another to form the final wipe assembly 10 shown in Figs. 1 and 2.

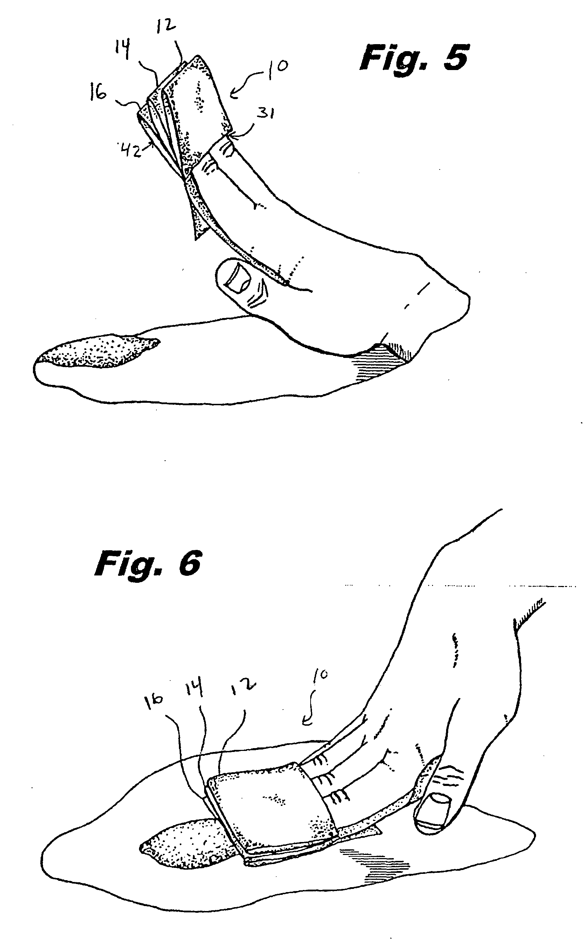

With reference to Figs. 5-8, a method of using the wipe assembly 10 according to the present invention will be described. First a user's hand is partially inserted within the chamber 31 such that the user's fingers are at least partially received within the chamber 31. In particular, the wipe assembly 10 is arranged such that the user's finger's are at least partially received within the chamber so that the remainder of the wipe assembly is maintained on the palm side of the user's hand. Once the user has inserted his or her finger's within the chamber 31 as shown in Fig. 5, the user may then utilize the bottom wipe 16 of the wipe assembly 10 to clean a surface, absorb a fluid, or use the wipe for some other cleaning purpose, as shown in Fig. 6. When the bottom wipe 16 is in its original stacked configuration it is noted that its second surface 42, i.e. its bottom surface is arranged in an outwardly facing configuration and thus the surface 42 can be utilized for cleaning purposes.

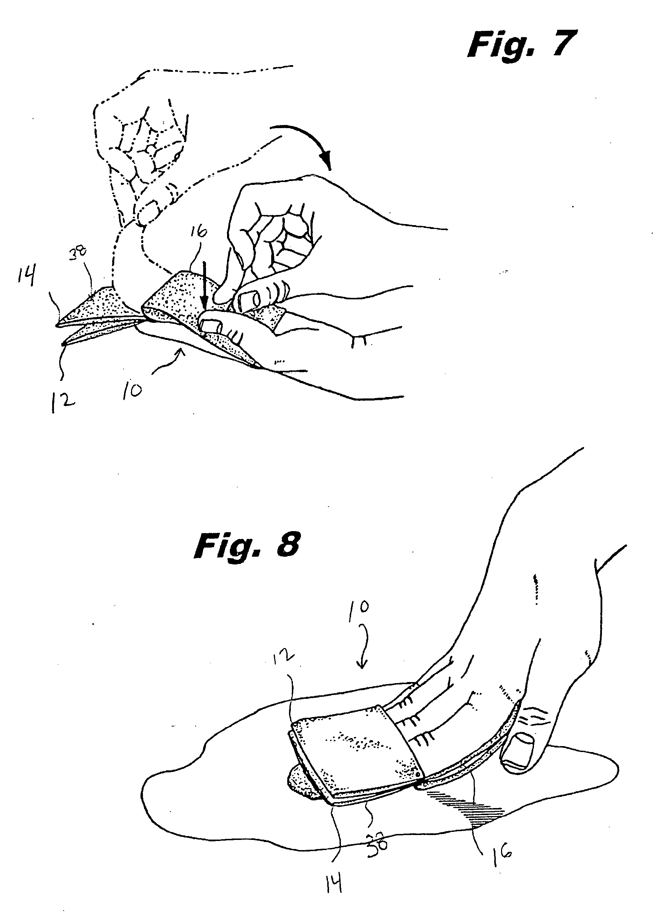

Referring to Fig. 7, after the user has utilized the bottom wipe 16, and desires a new clean wipe, the user may then rotate the bottom wipe 16 from its original stacked configuration shown in Figs. 5 and 6 to the position shown in Fig. 7. When the bottom wipe 16 is arranged in the position shown in Fig. 7 the user may retain the soiled bottom wipe 16 under the user's thumb to thereby expose the underlying wipe 14. Specifically, by retaining the bottom wipe 16 with the user's thumb as shown in Fig. 7, the second surface 38 of the intermediate wipe 14, i.e. its bottom surface, is arranged in an outwardly facing configuration and thus the surface 38 can be utilized for cleaning purposes. As shown in Fig. 8, during use the user can retain the soiled wipe 16 with his or her thumb and utilize the intermediate wipe 14 to further clean the soiled surface or the like. In this manner, the user does not have to substantially interrupt the cleaning process to access a new clean wipe. Rather, the user simple rotates the bottom wipe 16 from its original stacked configuration to its second position, retains the wipe 16 with his or her thumb, thereby exposing the intermediate wipe 14 and continues the cleaning process. After the intermediate wipe 14 is soiled the user may then likewise rotate the wipe 14 to thereby expose the top wipe 12 and continue the cleaning process.

Figure 4 depicts an exploded view of second embodiment of a wipe assembly 110 according to the present invention. The wipe assembly 110 generally includes a top wipe 112, a intermediate wipe 114, and a bottom wipe 116. Each of the intermediate and bottom wipes 114, 116 are arranged below the top wipe 112. Each of the wipes 112, 114 and 116 are coupled to one another and are arranged in a "stacked configuration" as shown in Fig. 4.

The specific embodiment of the wipe assembly 110 shown in Fig. 4 is constructed from three distinct sheets 118, 120 and 122 of material that are coupled to one another to thereby form the wipe assembly 110. The sheet 112 is folded to form to opposed material layers 124 and 126. The sheets of material 118, 120 and 122 may be coupled to one another in any known manner, for example by means of adhesive, sewn to one another, thermobonding or ultrasonic bonding (in which for either case, one or more of the layers is allowed to melt and resolidify so that it bonds with the other layer), selective hydroentanglement, or the like.

The layers 124 and 126 of the top wipe 112 are left in a detached state, i.e. they are not sealed to one another, along the respective proximal edges 129a and 129b thereof. In this manner, a chamber 131 is defined between the layers 124 and 126 of the top wipe 112. The chamber 131 is structured and arranged to receive at least a portion of a user's hand during use. More specifically, the chamber 131 is structured and arranged to receive at least a portion of the user's fingers. In this manner, a user can maintain the wipe assembly 110 on the palm side of the user's hand during use.

As shown in Fig. 4, the top wipe 112 includes a first surface 132 and a second opposed surface 134. Likewise, the intermediate wipe 114 includes opposed surfaces 136 and 138 and the bottom wipe 116 includes opposed surfaces 140 and 142.

The intermediate wipe 114 generally includes a main body portion 150 and a tab portion 152 that extends outwardly from the main body portion 150 of the intermediate wipe 114. Specifically, the tab portion 152 is structured and arranged such that it distally extends away from the main body portion 150, and the user, during use. Likewise, the bottom wipe 116 generally includes a main body portion 160 and a tab portion 162 that extends outwardly from the main body portion 160. Specifically, the tab portion 162 is structured and arranged such that it distally extends away from the main body portion 160, and the user, during use. As shown in Fig. 4, the tab 152 of the intermediate wipe 114 is preferably offset relative to the tab 162 of the bottom wipe 116, that is the tab 152 is not vertically aligned with the tab 162.

The embodiment of the wipe assembly 110 shown in Fig. 4 functions in use in the same manner as the first embodiment of the invention described above with referenced to Figures 1-3. That is, after the user has utilized the bottom wipe 116 to clean a surface or the like, and the user desires a new clean wipe, the user may rotate the bottom wipe 116 from its original stacked configuration towards the user and retain the soiled wipe with the user's thumb, thereby revealing the clean underlying intermediate wipe 114. The user may then use the intermediate wipe 114 to further clean the surface or the like. Thereafter, the user may then rotate the intermediate wipe 114 from its original stacked configuration towards the user and retain the soiled wipe with the user's thumb, thereby revealing the clean underlying top wipe 112. The tabs 152 and 162 are structured and arranged to enable the user to more easily grasp and rotate the bottom wipe 116 and the intermediate wipe 114 as such wipes are soiled during use.

Although the specific embodiment of the wipe assembly 110 shown in Fig. 4 includes three individual wipes, the wipe assembly 110 according to the present invention may include two or more individual wipes.

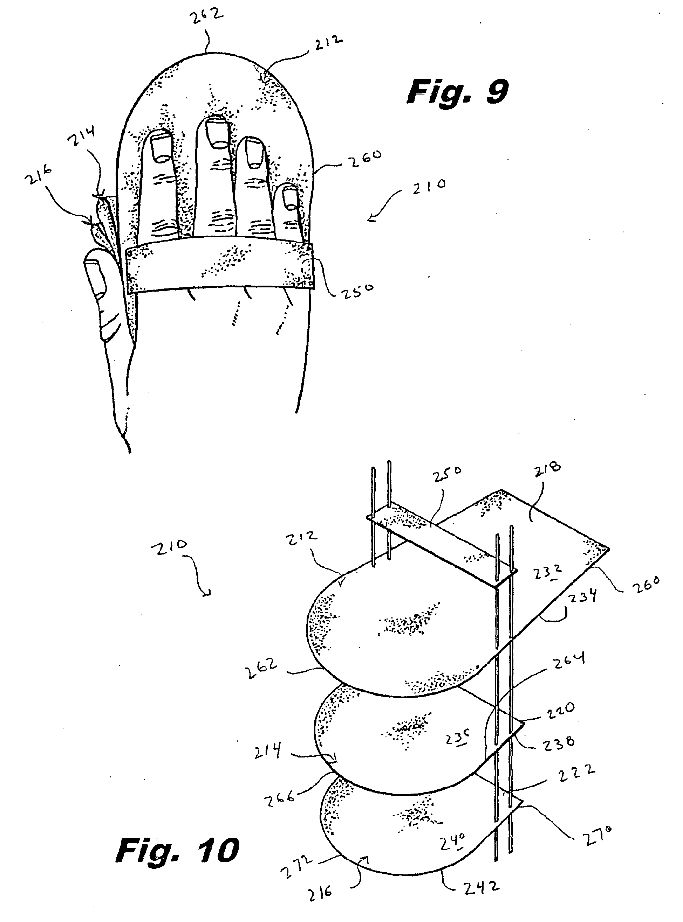

Figure 10 depicts an exploded view of third embodiment of a wipe assembly 210 according to the present invention. The wipe assembly 210 generally includes a top wipe 212, a intermediate wipe 214, and a bottom wipe 216. Each of the intermediate and bottom wipes 214, 216 are arranged below the top wipe 212. Each of the wipes 212, 214 and 216 are coupled to one another and are arranged in a "stacked configuration" as shown in Fig. 10.

The specific embodiment of the wipe assembly 210 shown in Figs. 9 and 10 is constructed from three distinct sheets 218, 220, and 222 of material that are coupled to one another to thereby form the wipe assembly 210. The sheets of material 218, 220 and 222 may be coupled to one another in any known manner, for example by means of adhesive, sewn to one another, thermobonding or ultrasonic bonding (in which for either case, one or more of the layers is allowed to melt and resolidify so that it bonds with the other layer), selective hydroentanglement, or the like.

As shown in Fig. 10, the top wipe 212 includes a first surface 232 and a second opposed surface 234. Likewise, the intermediate wipe 214 includes opposed surfaces 236 and 238 and the bottom wipe 216 includes opposed surfaces 240 and 242.

As shown in Fig. 9 and 10, the wipe assembly 210 further includes a strip 250 of material coupled to the top wipe 212 such that the strip 250 is arranged in opposed relationship to the first surface 232 of the top wipe 212. The strip 250 is structured and arranged so that a user may insert the user's hand between the strip 250 and the first surface 232 of the top wipe 212. In this manner, as shown in Fig. 9, a user can maintain the wipe assembly 210 on the palm side of the user's hand during use.

As shown in Figs. 9 and 10 the top wipe 212 is preferably dimensioned such that it extends from the base of a user's palm to the end of, or beyond, a user's fingertips. Stated another way, the top wipe 212 is preferably dimensioned such that the leading edge portion 262 of its peripheral edge 260 extends to or beyond a user's fingertips during use.

The embodiment of the wipe assembly 210 shown in Figs. 9 and 10 functions in use in the same manner as the first embodiment of the invention described above with referenced to Figures 1-3. That is, after the user has utilized the bottom wipe 216 to clean a surface or the like, and the user desires a new clean wipe, the user may rotate the bottom wipe 216 from its original stacked configuration towards the user and retain the soiled wipe with the user's thumb, thereby revealing the clean underlying intermediate wipe 214. The user may then use the intermediate wipe 214 to further clean the surface or the like. Thereafter, the user may then rotate the intermediate wipe 214 from its original stacked configuration towards the user and retain the soiled wipe with the user's thumb, thereby revealing the clean underlying top wipe 212.

The intermediate wipe 214 is preferably dimensioned such that the leading edge portion 266 of its peripheral edge 264 is recessed relative to the leading edge portion 272 of the bottom wipe's 216 peripheral edge 270. In a similar fashion, the top wipe 212 is preferably dimensioned such that the leading edge portion 262 of its peripheral edge 260 is recessed relative to the leading edge portion 266 of the intermediate wipe 214. This structure enables the user to more easily grasp the bottom wipe 216, and the intermediate wipe 214, as each wipe is soiled and rotate the respective wipe to reveal the underlying clean wipe.

The material sheets used to form the wipe assembly according to the present invention described herein may comprise a variety of both natural and synthetic fibers or materials. Nonlimiting examples of natural materials include, but are not limited to, silk fibers, keratin fibers, cellulosic fibers, and combinations thereof. Nonlimiting examples of synthetic materials include acetate fibers, acrylic fibers, cellulose ester fibers, modacrylic fibers, polamide fibers, polyester fibers, rayon fibers, and combinations thereof. Preferably, the material sheets used to form the wipe assembly according to the present invention comprise a nonwoven material formed from one or more of the above identified fiberous materials.

## Claims

1. A wipe assembly (10; 110; 210) comprising:
a plurality of wipes (14, 16; 114, 116; 214, 216) arranged in a stacked configuration, each one of the wipes having a first surface and an opposed second surface; and
a mechanism (31; 131; 250) for maintaining the wipe assembly on a palm side of a user's hand during use of the wipe assembly;
each one of the wipes being capable of movement from a first position to a second position, wherein in the first position the second surface of the wipe is arranged in an outwardly facing configuration and in the second position the second surface of an underlying wipe is arranged in an outwardly facing configuration;
**characterized in that** each wipe (14, 16; 114, 116; 214, 216) is structured and arranged such that the wipe is rotatable from the first position to the second position and may be manually retained in the second position by a user when the wipe assembly (10; 110; 210) is maintained by the mechanism (31; 131; 250) on the palm side of the user's hand.

2. The wipe assembly of claim 1, wherein the plurality of wipes comprises:
a top wipe (12) having a first surface (32) and an opposed second surface (34);
a bottom wipe (16) having a first surface (40) and an opposed second surface (42), and
an intermediate wipe (14) arranged between the top wipe and the bottom wipe, the intermediate wipe having a first surface (36) and an opposed second surface (38);
wherein the top wipe, the intermediate wipe, and the bottom wipe are coupled to one another and have a first position wherein the wipes are arranged in a stacked configuration;
wherein the bottom wipe is capable of rotation from the first position to a second position, wherein in the first position the second surface of the bottom wipe is arranged in an outwardly facing configuration and in the second position the second surface of the intermediate wipe is arranged in an outwardly facing configuration; and
wherein the intermediate wipe is capable of rotation from the first position to a second position, wherein in the first position the second surface of the intermediate wipe is arranged in an outwardly facing configuration and in the second position the second surface of the top wipe is arranged in an outwardly facing configuration,
wherein each of the bottom and intermediate wipes are structured and arranged such that the bottom and intermediate wipes may be manually retained in the second position by a user when the wipe assembly is maintained by the mechanism on the palm side of the user's hand.

3. The wipe assembly according to claim 1, wherein a top wipe of the plurality of wipes, or the wipe assembly according to claim 2, wherein the top wipe, comprises a first layer (18) and a second layer (20) of material.

4. The wipe assembly according to claim 3, wherein the first and second layers are coupled to one another to define a chamber (31) between the layers, the chamber being the mechanism for maintaining the wipe assembly on a palm side of the user's hand during use of the wipe assembly.

5. The wipe assembly according to claim 4, wherein the wipe assembly (10) is formed from a single folded sheet of material.

6. The wipe assembly according to claim 1, wherein a sheet of material forms a top wipe (12) of the plurality of wipes, the sheet being folded to define first (18) and second (20) opposed layers of material, or the wipe assembly according to claim 2, wherein the top wipe (12) is formed by a folded sheet defining first (18) and second (20) opposed layers of material, the first and second layers being coupled to one another to define a chamber (31) between the layers, the chamber being the mechanism for maintaining the wipe assembly on a palm side of the user's hand during use of the wipe assembly.

7. The wipe assembly according to claim 1 or 2, wherein the mechanism comprises a sheet of flexible material coupled to the plurality of wipes or the top wipe, the sheet of material being structured and arranged to permit a user to insert at least part of the user's hand between the sheet of material and a top wipe of the plurality of wipes or the top wipe to thereby maintain the wipe assembly on a palm side of the user's hand during use of the wipe assembly.

8. The wipe assembly according to claim 1, wherein each wipe is structured and arranged such that the wipe may be maintained in the second position by a user's thumb when the wipe assembly is maintained on the palm side of the user's hand.

9. The wipe assembly according to claim 1, wherein the plurality of wipes includes at least a first wipe and a second wipe each of the first wipe and the second wipe including, or the wipe assembly according to claim 2, wherein each of intermediate wipe and bottom wipe include, a main body portion and a tab portion (152, 162) extending outwardly from the main body portion.

10. The wipe assembly according claim 9, wherein each tab portion is structured and arranged such that it extends distally away from a user during use of the wipe assembly.

11. The wipe assembly according to claim 10, wherein the tab portion (152) of the first wipe is offset relative to the tab portion (162) of the second wipe.

12. The wipe assembly according to claim 2, wherein the top wipe, intermediate wipe, and bottom wipe include a peripheral edge, and wherein the peripheral edge of the top wipe, intermediate wipe, and bottom wipe each includes a leading edge portion, the leading edge portion of the top wipe being recessed relative to the leading edge portion of the intermediate wipe, and the leading edge portion of the intermediate wipe being recessed relative to the leading edge portion of the bottom wipe.

13. A method of cleaning comprising the steps of:
maintaining a wipe assembly (10; 110; 210) on a palm side of a user's hand, the wipe assembly including a plurality of wipes (12, 14, 16; 112, 114; 116; 212, 214, 216) arranged in a stacked configuration, each one of the wipes having a first and second opposed surfaces;
cleaning a surface with a bottom wipe (14; 114; 214) of the plurality of wipes;
moving the bottom wipe from a first position to a second position, wherein in the first position the second surface (42) of the wipe is arranged in an outwardly facing configuration and in the second position the second surface (38) of an underlying wipe is arranged in an outwardly facing configuration; and
cleaning a surface with the underlying wipe;
**characterized in that** the bootom wipe is rotated from the first position to the second position and the method further comprises manually retaining the bottom wipe (14; 114; 214) in the second position.

14. The method according to claim 13, wherein the step of maintaining the wipe assembly on a palm side of the user's hand comprises at least partially inserting a user's fingers into a mechanism (31; 131; 250) for maintaining the plurality of wipes on a palm side of a user's hand.

15. The method according to claim 14, wherein the step of manually retaining the bottom wipe in the second position comprises retaining the bottom wipe in the second position by means of a user's thumb.

16. The method according to claim 15, further comprising rotating the underlying wipe (14) from a first position to a second position, wherein in the first position the second surface (38) of the underlying wipe is arranged in an outwardly facing configuration and in the second position the second surface (34) of a second underlying wipe (12) is arranged in an outwardly facing configuration.

17. The method of claim 13, wherein the plurality of wipes includes a top wipe (12), a bottom wipe (16), and an intermediate wipe (14) arranged between the top and bottom wipe, each of the wipes having a first position wherein the wipe is arranged in a stacked configuration relative to the other wipes, and wherein the step of cleaning a surface with the underlying wipe comprises cleaning a surface with the intermediate wipe.

18. The method according to claim 17, further comprising rotating the intermediate wipe from a first position to a second position, wherein in the first position the second surface of the intermediate wipe is arranged in an outwardly facing configuration and in the second position the second surface of the top wipe is arranged in an outwardly facing configuration.

19. The method according to claim 18, further comprising cleaning a surface with the top wipe.

## Patentansprüche

1. Wischeinheit (10; 110; 210), umfassend:
mehrere Wischvorrichtungen (14, 16; 114, 116; 214, 216), die in einer gestapelten Konfiguration angeordnet sind, wobei jede Wischvorrichtung eine erste Oberfläche und eine gegenüberliegende zweite Oberfläche aufweist; und
einen Mechanismus (31; 131; 250) zum Halten der Wischeinheit an einer Handinnenseite einer Benutzerhand während der Verwendung der Wischeinheit;
wobei sich jede der Wischvorrichtungen von einer ersten Position zu einer zweiten Position bewegen kann, wobei in der ersten Position die zweite Oberfläche der Wischvorrichtung in einer nach außen gerichteten Konfiguration angeordnet ist und wobei in der zweiten Position die zweite Oberfläche einer darunterliegenden Wischvorrichtung in einer nach außen gerichteten Konfiguration angeordnet ist;
**dadurch gekennzeichnet, dass**
jede Wischvorrichtungen (14, 16; 114, 116; 214, 216) derart strukturiert und angeordnet ist, dass die Wischvorrichtung von der ersten Position zu der zweiten Position drehbar ist und manuell von einem Benutzer in der zweiten Position gehalten werden kann, wenn die Wischeinheit (10; 110; 210) von dem Mechanismus (31; 131; 250) an der Handinnenseite der Benutzerhand gehalten wird.

2. Wischeinheit nach Anspruch 1, wobei die mehreren Wischvorrichtungen Folgendes umfassen:
eine obere Wischvorrichtung (12) mit einer ersten Oberfläche (32) und einer gegenüberliegenden zweiten Oberfläche (34);
eine untere Wischvorrichtung (16) mit einer ersten Oberfläche (40) und einer gegenüberliegenden zweiten Oberfläche (42) und einer dazwischen liegenden Wischvorrichtung (14), die zwischen der oberen Wischvorrichtung und der unteren Wischvorrichtung angeordnet ist, wobei die dazwischen liegende Wischvorrichtung eine erste Oberfläche (36) und eine gegenüberliegende zweite Oberfläche (38) aufweist;
wobei die obere Wischvorrichtung, die dazwischen liegende Wischvorrichtung und die untere Wischvorrichtung miteinander gekoppelt sind und eine erste Position aufweisen, in der die Wischvorrichtungen in einer gestapelten Konfiguration angeordnet sind;
wobei sich die untere Wischvorrichtung von der ersten Position zu einer zweiten Position drehen kann, wobei in der ersten Position die zweite Oberfläche der unteren Wischvorrichtung in einer nach außen gerichteten Konfiguration angeordnet ist und wobei in der zweiten Position die zweite Oberfläche der dazwischen liegenden Wischvorrichtung in einer nach außen gerichteten Konfiguration angeordnet ist; und
wobei sich die dazwischen liegende Wischvorrichtung von der ersten Position zu einer zweiten Position drehen kann, wobei in der ersten Position die zweite Oberfläche der dazwischen liegenden Wischvorrichtung in einer nach außen gerichteten Konfiguration angeordnet ist und wobei in der zweiten Position die zweite Oberfläche der oberen Wischvorrichtung in einer nach außen gerichteten Konfiguration angeordnet ist,
wobei jede der unteren und dazwischen liegenden Wischvorrichtungen derart strukturiert und angeordnet ist, dass die untere und dazwischen liegende Wischvorrichtung manuell von einem Benutzer in der zweiten Position gehalten werden können, wenn die Wischeinheit von dem Mechanismus an der Handinnenseite der Benutzerhand gehalten wird.

3. Wischeinheit nach Anspruch 1, wobei eine obere Wischvorrichtung der mehreren Wischvorrichtungen, oder Wischeinheit nach Anspruch 2, wobei die obere Wischvorrichtung eine erste Schicht (18) und eine zweite Schicht (20) aus Material umfasst.

4. Wischeinheit nach Anspruch 3, wobei die erste und die zweite Schicht miteinander gekoppelt sind, um eine Kammer (31) zwischen den Schichten zu definieren, wobei die Kammer der Mechanismus zum Halten der Wischeinheit an der Handinnenseite der Benutzerhand während der Verwendung der Wischeinheit ist.

5. Wischeinheit nach Anspruch 4, wobei die Wischeinheit (10) aus einer einzelnen gefalteten Materialbahn hergestellt ist.

6. Wischeinheit nach Anspruch 1, wobei eine Materialbahn eine obere Wischvorrichtung (12) der mehreren Wischvorrichtungen bildet, wobei die Bahn zum Definieren einer ersten (18) und einer zweiten (20) gegenüberliegenden Materialschicht gefaltet wird, oder Wischeinheit nach Anspruch 2, wobei die obere Wischvorrichtung (12) von einer gefalteten Bahn gebildet wird, welche die erste (18) und die zweite (20) gegenüberliegende Materialschicht definiert, wobei die erste und die zweite Schicht miteinander gekoppelt sind, um eine Kammer (31) zwischen den Schichten zu definieren, wobei die Kammer der Mechanismus zum Halten der Wischeinheit an der Handinnenseite der Benutzerhand während der Verwendung der Wischeinheit ist.

7. Wischeinheit nach Anspruch 1 oder 2, wobei der Mechanismus eine Bahn aus flexiblem Material umfasst, das mit mehreren Wischvorrichtungen oder der oberen Wischvorrichtung gekoppelt ist, wobei die Materialbahn derart strukturiert und angeordnet ist, dass der Benutzer mindestens einen Teil der Benutzerhand zwischen die Materialbahn und die obere Wischvorrichtung der mehreren Wischvorrichtungen oder der oberen Wischvorrichtung einführen kann, wodurch die Wischeinheit während der Verwendung der Wischeinheit an einer Handinnenseite der Benutzerhand gehalten wird.

8. Wischeinheit nach Anspruch 1, wobei jede Wischvorrichtung derart strukturiert und angeordnet ist, dass die Wischvorrichtung mithilfe des Benutzerdaumens in der zweiten Position gehalten werden kann, wenn die Wischeinheit an der Handinnenseite des Benutzers gehalten wird.

9. Wischeinheit nach Anspruch 1, wobei die mehreren Wischvorrichtungen mindestens eine erste Wischvorrichtung und eine zweite Wischvorrichtung aufweisen, wobei sowohl die erste Wischvorrichtung als auch die zweite Wischvorrichtung oder die Wischeinheit nach Anspruch 2, wobei jede der dazwischen liegenden Wischvorrichtung und untere Wischvorrichtung einen Hauptkörperabschnitt und einen Laschenabschnitt (152, 162) aufweist, der sich von dem Hauptkörperabschnitt, nach außen erstreckt.

10. Wischeinheit nach Anspruch 9, wobei jeder Laschenabschnitt derart strukturiert und angeordnet ist, um sich während der Verwendung der Wischeinheit distal von einem Benutzer zu erstrecken.

11. Wischeinheit nach Anspruch 10, wobei der Laschenabschnitt (152) der ersten Wischvorrichtung in Bezug auf den Laschenabschnitt (162) der zweiten Wischvorrichtung versetzt ist.

12. Wischeinheit nach Anspruch 2, wobei die obere Wischvorrichtung, die dazwischen liegende Wischvorrichtung und die untere Wischvorrichtung einen Umfangsrand aufweisen, und wobei der Umfangsrand der oberen Wischvorrichtung, der dazwischen liegenden Wischvorrichtung und der unteren Wischvorrichtung jeweils einen Vorderkantenabschnitt aufweisen, wobei der Vorderkantenabschnitt der oberen Wischvorrichtung in Bezug auf den Vorderkantenabschnitt der dazwischen liegenden Wischvorrichtung eingesenkt ist und wobei der Vorderkantenabschnitt der dazwischen liegenden Wischvorrichtung in Bezug auf den Vorderkantenabschnitt der unteren Wischvorrichtung eingesenkt ist.

13. Reinigungsverfahren, umfassend die folgenden Schritte:
Halten einer Wischeinheit (10; 110; 210) an einer Handinnenseite einer Benutzerhand, wobei die Wischeinheit mehrere Wischvorrichtungen (12, 14, 16; 112, 114; 116; 212, 214, 216) aufweist, die in einer gestapelten Konfiguration angeordnet sind, wobei jede der Wischvorrichtungen eine erste und eine zweite gegenüberliegende Oberfläche aufweist;
Reinigen einer Oberfläche mit einer unteren Wischvorrichtung (14; 114; 214) der mehreren Wischvorrichtungen;
Bewegen der unteren Wischvorrichtung von einer ersten zu einer zweiten Position, wobei in der ersten Position die zweite Oberfläche (42) der Wischvorrichtung in einer nach außen gerichteten Konfiguration und in der zweiten Position die zweite Oberfläche (38) einer darunterliegenden Wischvorrichtung in einer nach außen gerichteten Konfiguration ausgerichtet ist; und
Reinigen einer Oberfläche mit der darunterliegenden Wischvorrichtung; **dadurch gekennzeichnet, dass** die untere Wischvorrichtung von der ersten Position zu der zweiten Position gedreht wird und das Verfahren ferner das manuelle Halten der unteren Wischvorrichtung (14; 114; 214) in der zweiten Position umfasst.

14. Verfahren nach Anspruch 13, wobei der Schritt des Haltens der Wischeinheit an der Handinnenseite der Benutzerhand mindestens das Einführen von Benutzerfingern in den Mechanismus (31; 131; 250) zum Halten der mehreren Wischvorrichtungen an einer Handinnenseite einer Benutzerhand umfasst.

15. Verfahren nach Anspruch 14, wobei der Schritt des manuellen Haltens der unteren Wischvorrichtung mithilfe eines Benutzerdaumens in der zweiten Position das Halten der unteren Wischvorrichtung in der zweiten Position umfasst.

16. Verfahren nach Anspruch 15, ferner umfassend das Drehen der darunterliegenden Wischvorrichtung (14) von einer ersten Position zu einer zweiten Position, wobei in der ersten Position die zweite Oberfläche (38) der darunterliegenden Wischvorrichtung in einer nach außen gerichteten Konfiguration und in der zweiten Position die zweite Oberfläche (34) einer zweiten darunterliegenden Wischvorrichtung (12) in einer nach außen gerichteten Konfiguration angeordnet ist.

17. Verfahren nach Anspruch 13, wobei die mehreren Wischvorrichtungen eine obere Wischvorrichtung (12), eine untere Wischvorrichtung (16) und eine dazwischen liegende Wischvorrichtung (14), die zwischen der oberen und unteren Wischvorrichtung angeordnet ist, aufweisen, wobei jede Wischvorrichtung einen ersten Abschnitt aufweist und wobei die Wischvorrichtung in einer gestapelten Konfiguration in Bezug auf die anderen Wischvorrichtungen angeordnet ist, und wobei der Schritt des Reinigens einer Oberfläche mit der darunterliegenden Wischvorrichtung das Reinigen einer Oberfläche mit der dazwischen liegenden Wischvorrichtung umfasst.

18. Verfahren nach Anspruch 17, ferner umfassend das Drehen der dazwischen liegenden Wischvorrichtung von einer ersten Position zu einer zweiten Position, wobei in der ersten Position die zweite Oberfläche der dazwischen liegenden Wischvorrichtung in einer nach außen gerichteten Konfiguration und in der zweiten Position die zweite Oberfläche einer oberen Wischvorrichtung in einer nach außen gerichteten Konfiguration angeordnet ist.

19. Verfahren nach Anspruch 18, ferner umfassend das Reinigen einer Oberfläche mit der oberen Wischvorrichtung.

## Revendications

1. Assemblage de lingettes (10 ; 110 ; 21), comprenant :
une pluralité de lingettes (14, 16 ; 114, 116 ; 214, 216) agencées suivant une configuration empilée, chacune des lingettes ayant une première surface et une deuxième surface opposée ; et
un mécanisme (31 ; 131 ; 250) destiné à maintenir l'assemblage de lingettes sur un côté paume d'une main d'un utilisateur lors de l'utilisation de l'assemblage de lingettes ;
chacune des lingettes étant apte à être déplacée d'une première position à une deuxième position,
la deuxième surface de la lingette, dans la première position, étant agencée dans une configuration tournée vers l'extérieur et, dans la deuxième position, la deuxième surface d'une lingette sous-jacente étant agencée dans une configuration tournée vers l'extérieur ;
**caractérisé en ce que** chaque lingette (14, 16 ; 114, 116 ; 214, 216) est structurée et agencée de telle sorte que la lingette puisse tourner de la première position à la deuxième position et puisse être manuellement retenue dans la deuxième position par un utilisateur lorsque l'assemblage de lingettes (10 ; 110 ; 210) est maintenu par le mécanisme (31 ; 131 ; 250) sur le côté paume de la main de l'utilisateur.

2. Assemblage de lingettes selon la revendication 1, dans lequel la pluralité de lingettes comprend :
une lingette supérieure (12) ayant une première surface (32) et une deuxième surface opposée (34) ;
une lingette inférieure (16) ayant une première surface (40) et une deuxième surface opposée (42), et
une lingette intermédiaire (14) agencée entre la lingette supérieure et la lingette inférieure, la lingette intermédiaire ayant une première surface (36) et une deuxième surface opposée (38) ;
la lingette supérieure, la lingette intermédiaire et la lingette inférieure étant accouplées les unes aux autres et ayant une première position dans laquelle les lingette sont agencées dans une configuration empilée ;
la lingette inférieure étant capable de tourner de la première position à une deuxième position, la deuxième surface de la lingette inférieure, dans la première position, étant agencée dans une configuration tournée vers l'extérieur, et dans la deuxième position, la deuxième surface de la lingette intermédiaire étant agencée dans une configuration tournée vers l'extérieur ; et
la lingette intermédiaire étant apte à être tournée de la première position à une deuxième position, la deuxième surface de la lingette intermédiaire, dans la première position, étant agencée dans une configuration tournée vers l'extérieur et dans la deuxième position, la deuxième surface de la lingette supérieure étant agencée dans une configuration tournée vers l'extérieur,
chacune des lingettes inférieure et intermédiaire étant structurée et agencée de telle sorte que les lingettes inférieure et intermédiaire puissent être retenues manuellement dans la deuxième position par un utilisateur lorsque l'assemblage de lingettes est maintenu par le mécanisme sur le côté paume de la main de l'utilisateur.

3. Assemblage de lingettes selon la revendication 1, dans lequel une lingette supérieure de la pluralité de lingettes, ou assemblage de lingettes selon la revendication 2, dans lequel la lingette supérieure, comprend une première couche (18) et une deuxième couche (20) de matériau.

4. Assemblage de lingettes selon la revendication 3, dans lequel les première et deuxièmes couches sont accouplées l'une à l'autre pour définir une chambre (31) entre les couches, la chambre constituant le mécanisme pour maintenir l'assemblage de lingettes sur un côté paume de la main de l'utilisateur au cours de l'utilisation de l'assemblage de lingettes.

5. Assemblage de lingettes selon la revendication 4, dans lequel l'assemblage de lingettes (10) est formé d'une feuille de matériau pliée unique.

6. Assemblage de lingettes selon la revendication 1, dans lequel une feuille de matériau forme une lingette supérieure (12) de la pluralité de lingettes, la feuille étant pliée pour définir des première (18) et deuxième (20) couches de matériau opposées, ou assemblage de lingettes selon la revendication 2, dans lequel la lingette supérieure (12) est formée par une feuille pliée définissant des première (18) et deuxième (20) couches de matériau opposées, les première et deuxième couches étant accouplées l'une à l'autre pour définir une chambre (31) entre les couches, la chambre constituant le mécanisme pour maintenir l'assemblage de lingettes sur un côté paume de la main de l'utilisateur au cours de l'utilisation de l'assemblage de lingettes.

7. Assemblage de lingettes selon la revendication 1 ou 2, dans lequel le mécanisme comprend une feuille de matériau flexible accouplé à la pluralité de lingettes ou à la lingette supérieure, la feuille de matériau étant structurée et agencée de manière à permettre à un utilisateur d'insérer au moins une partie de la main de l'utilisateur entre la feuille de matériau et une lingette supérieure de la pluralité de lingettes ou la lingette supérieure pour ainsi maintenir l'assemblage de lingettes sur un côté paume de la main de l'utilisateur au cours de l'utilisation de l'assemblage de lingettes.

8. Assemblage de lingettes selon la revendication 1, dans lequel chaque lingette est structurée et agencée de telle sorte que la lingette puisse être maintenue dans la deuxième position par un pouce de l'utilisateur lorsque l'assemblage de lingettes est maintenu sur le côté paume de la main de l'utilisateur.

9. Assemblage de lingettes selon la revendication 1, dans lequel la pluralité de lingettes comporte au moins une première lingette et une deuxième lingette, chacune de la première lingette et de la deuxième lingette comportant, ou assemblage de lingettes selon la revendication 2, dans lequel chacune de la lingette intermédiaire et de la lingette inférieur comporte, une portion de corps principale et une portion de languette (152, 162) s'étendant vers l'extérieur à partir de la portion de corps principale.

10. Assemblage de lingettes selon la revendication 9, dans lequel chaque portion de languette est structurée et agencée de telle sorte qu'elle s'étende distalement à l'écart d'un utilisateur au cours de l'utilisation de l'assemblage de lingettes.

11. Assemblage de lingettes selon la revendication 10, dans lequel la portion de languette (152) de la première lingette est décalée par rapport à la portion de languette (162) de la deuxième lingette.

12. Assemblage de lingettes selon la revendication 2, dans lequel la lingette supérieure, la lingette intermédiaire et la lingette inférieure comportent un bord périphérique et dans lequel le bord périphérique de la lingette supérieure, de la lingette intermédiaire et de la lingette inférieure comporte à chaque fois une portion de bord avant, la portion de bord avant de la lingette supérieure étant en retrait par rapport à la portion de bord avant de la lingette intermédiaire, et la portion de bord avant de la lingette intermédiaire étant en retrait par rapport à la portion de bord avant de la lingette inférieure.

13. Procédé de nettoyage comprenant les étapes suivantes :
maintenir un assemblage de lingettes (10 ; 110 ; 210) sur un côté paume d'une main d'un utilisateur, l'assemblage de lingettes comportant une pluralité de lingettes (12, 14, 16 ; 112, 114, 116 ; 212, 214, 216) agencée dans une configuration empilée, chacune des lingettes ayant une première et une deuxième surface opposée ;
nettoyer une surface avec une lingette inférieure (14 ; 114 ; 214) de la pluralité de lingettes ;
déplacer la lingette inférieure d'une première position à une deuxième position, la deuxième surface (42) de la lingette, dans la première position, étant agencée dans une configuration tournée vers l'extérieur et dans la deuxième position, la deuxième surface (38) d'une lingette sous-jacente étend agencée dans une configuration tournée vers l'extérieur ; et
nettoyer une surface avec la lingette sous-jacente ;
**caractérisé en ce que** la lingette inférieure est tournée de la première position à la deuxième position et le procédé comprend en outre de retenir manuellement la lingette inférieure (14 ; 114 ; 214) dans la deuxième position.

14. Procédé selon la revendication 13, dans lequel l'étape consistant à maintenir l'assemblage de lingettes sur un côté paume de la main de l'utilisateur comprend d'insérer au moins en partie les doigts d'un utilisateur dans un mécanisme (31 ; 131 ; 250) pour maintenir la pluralité de lingettes sur un côté paume d'une main d'un utilisateur.

15. Procédé selon la revendication 14, dans lequel l'étape de retenir manuellement la lingette inférieure dans la deuxième position comprend de retenir la lingette inférieure dans la deuxième position au moyen d'un pouce d'un utilisateur.

16. Procédé selon la revendication 15, comprenant en outre la rotation de la lingette sous-jacente (14) d'une première position à une deuxième position, la deuxième surface (38) de la lingette sous-jacente, dans la première position, étant agencée dans une configuration tournée vers l'extérieur et dans la deuxième position, la deuxième surface (34) d'une deuxième lingette sous-jacente (12) étant agencée dans une configuration tournée vers l'extérieur.

17. Procédé selon la revendication 13, dans lequel la pluralité de lingettes comporte une lingette supérieure (12), une lingette inférieure (16), et une lingette intermédiaire (14) agencée entre la lingette supérieure et la lingette inférieure, chacune des lingettes ayant une première position dans laquelle la lingette est agencée dans une configuration empilée par rapport aux autres lingettes, et dans lequel l'étape de nettoyage d'une surface avec la lingette sous-jacente comprend le nettoyage d'une surface avec la lingette intermédiaire.

18. Procédé selon la revendication 17, comprenant en outre la rotation de la lingette intermédiaire d'une première position à une deuxième position, la deuxième surface de la lingette intermédiaire, dans la première position, étant agencée dans une configuration tournée vers l'extérieur et dans la deuxième position, la deuxième surface de la lingette supérieure étant agencée dans une configuration tournée vers l'extérieur.

19. Procédé selon la revendication 18, comprenant en outre le nettoyage d'une surface avec la lingette supérieure.
